(19) Europäisches Patentamt
European Patent Office
Office européen des brevets

(11) **EP 2 796 153 A1**

(12) **EUROPEAN PATENT APPLICATION**
published in accordance with Art. 153(4) EPC

(43) Date of publication:
**29.10.2014 Bulletin 2014/44**

(21) Application number: **13771761.7**

(22) Date of filing: **01.04.2013**

(51) Int Cl.:
***A61K 49/06*** *(2006.01)* ***A61K 9/16*** *(2006.01)*
***A61K 9/14*** *(2006.01)*

(86) International application number:
**PCT/KR2013/002666**

(87) International publication number:
**WO 2013/151283 (10.10.2013 Gazette 2013/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.04.2012 KR 20120035821**

(71) Applicant: **SNU R&DB Foundation**
**Seoul 151-742 (KR)**

(72) Inventors:
- **HYEON, Taeghwan**
  **Seoul 135-537 (KR)**
- **LEE, Nohyun**
  **Daejeon 302-901 (KR)**
- **CHOI, Seunghong**
  **Seoul 158-051 (KR)**

(74) Representative: **Bassil, Nicholas Charles et al**
**Kilburn & Strode LLP**
**20 Red Lion Street**
**London WC1R 4PJ (GB)**

(54) **IRON OXIDE NANOCOMPOSITE, MAGNETIC RESONANCE IMAGING T2 CONTRAST MEDIUM COMPRISING SAME, AND METHOD FOR MANUFACTURING SAME**

(57) The present invention relates to iron oxide nanocomposite, magnetic resonance imaging T2 contrast medium compring same, and method for manufacturing same. More particularly, the present invention is directed to An MRI (magnetic resonance imaging) T2 contrast agent, comprising an iron oxide nanocomposite which includes an iron oxide nanoparticle, wherein said iron oxide nanoparticle is encapsulated with a surfactant and said surfactant is encapsulated with polyethylene glycol-phospholipid and method for preparing the same.

Fig. 8

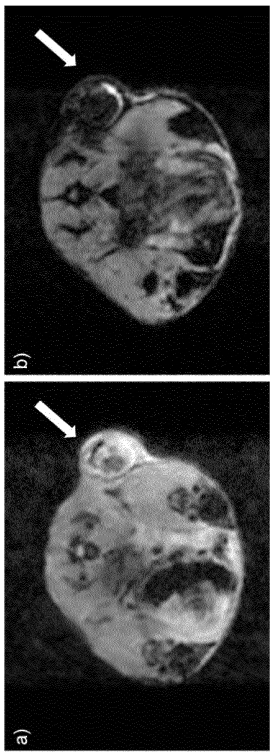


EP 2 796 153 A1

## Description

### Technical Field

The present invention relates to iron oxide nanocomposite, magnetic resonance imaging $T_2$ contrast agent compring the same, and method for manufacturing the same. More particularly, the present invention is directed to an iron oxide nanocomposite comprising an iron oxide nanoparticle, wherein the iron oxide nanoparticle is encapsulated with a surfactant and the surfactant is encapsulated with polyethylene glycol-phospholipid, an MRI $T_2$ contrast agent comprising the same, and method for preparing the same.

### Background Art

Magnetic nanoparticles have received enormous attention in various research areas because of their unique magnetic properties, facile surface modification, and biocompatibility. Magnetic nanoparticles have been used as contrast agents for magnetic resonance imaging (MRI) [Y.-w. Jun, J.-H. Lee, J. Cheon, Angew. Chem. 2008, 120, 5200; Angew. Chem. Int. Ed. 2008, 47, 5122; H. B. Na, I. C. Song, T. Hyeon, Adv. Mater. 2009, 21, 2133; J. Xie, G. Liu, H. S. Eden, H. Ai, X. Chen, Acc. Chem. Res. 2011, 44, 883; M. Srinivas, E. H. J. G. Aarntzen, J. W. M. Bulte, W. J. Oyen, A. Heerschap, I. J. M. de Vries, C. G. Figdor, Adv. Drug. Deliv. Rev. 2010, 62, 1080; N. Lee, T. Hyeon. Chem. Soc. Rev. 2012, DOI: 10.1039/c1cs15248c], drug delivery vehicles, hyperthermia agents, and for magnetic separation.

Among them, MRI is one of the key applications of magnetic nanoparticles because the inherently low sensitivity of MRI can be improved by magnetic nanoparticles. Magnetic nanoparticles accelerate spin-spin relaxation of adjacent protons by producing a magnetic field, resulting in signal attenuation in $T_2$-weighted MR images.

Among the various magnetic nanomaterials available, iron oxide nanoparticles have been most widely used as $T_2$ MRI contrast agents since they are biologically well tolerated and benign [P. Bourrinet, H. H. Bengele, B. Bonnemain, A. Dencausse, J.-M. Idee, P. M. Jacobs, J. M. Lewis, Invest. Radiol. 2006, 41, 313; T. K. Jain, M. K. Reddy, M. A. Morales, D. L. Leslie-Pelecky, V. Labhasetwar, Mol. Pharm. 2008, 5, 316].

Several iron oxide nanoparticle-based MRI contrast agents including Feridex and Resovist have been approved for clinical use. However, these iron oxide nanoparticles, which are prepared by coprecipitation, are relatively polydisperse, and their magnetic property and relaxivity are difficult to control.

Owing to significant recent advances in synthetic methods for nanocrystals, including the well-known thermal decomposition process, uniform and highly crystalline iron oxide nanocrystals with sizes ranging from a few nanometers to tens of nanometers are now available for various medical applications.

Since the $T_2$ contrast effect of iron oxide nanoparticles is dependent on their magnetic moment, which is proportional to their volume, size control of nanoparticles is critical for achieving high relaxivity.

A previous report shows that the $r_2$ relaxivity of superparamagnetic iron oxide nanoparticles increased with the size of the nanoparticles [Y.-w. Jun, Y.-M. Huh, J.-s. Choi, J.-H. Lee, H.-T. Song, S. Kim, S. Yoon, K.-S. Kim, J.-S. Shin, J.-S. Suh, J. Cheon J. Am. Chem. Soc. 2005, 127, 5732]. For magnetic nanoparticles smaller than 30 nm, fast diffusion averages the magnetic field due to iron oxide nanoparticles (motional averaging regime, MAR) [R. A. Brooks, F. Moiny, P. Gillis, Magn. Reson. Med. 2001, 45, 1014; R. A. Brooks. Magn. Reson. Med. 2002, 47, 388]. In this MAR, the relaxivity of nanoparticles is dependent on their size.

For large-sized nanoparticles, the diffusion effect becomes small, and nanoparticles can be regarded as randomly distributed stationary objects (static dephasing regime, SDR). In the SDR, nanoparticles are predicted to exhibit the highest $r_2$ relaxivity, which is independent of their size.

However, iron oxide nanoparticles in the SDR are ferrimagnetic, and their magnetic dipole interaction results in poor colloidal stability [D. Kim, N. Lee, M. Park, B. H. Kim, K. An, T. Hyeon, J. Am. Chem. Soc. 2009, 131, 454; N. Lee et al., Proc. Natl. Acad. Sci. USA 2011, 108, 2662]. The severe agglomeration of ferrimagntic iron oxide nanoparticles precludes their in vivo applications because their circulation time is very short, and they can sometimes cause organ damage because of capillary occlusion [P. Moroz, C. Metcalf, B. N. Gray, Biometals 2003, 16, 455].

An alternative method to avoid ferrimagnetic dipole interaction is controlled clustering of multiple superparamagnetic iron oxide nanoparticles using larger templates such as silica and polymers since the T2 contrast effect also increases with an increasing number of nanoparticles in the aggregates [T.-J. Yoon, H. Lee, H. Shao, S. A. Hilderbrand, R. Weissleder, Adv. Mater. 2011, 23, 4793 and its Supporting Information; J.-H. Lee, Y.-w. Jun, S.-I. Yeon, J.-S. Shin, J. Cheon, Angew. Chem. 2006, 118, 8340; Angew. Chem. Int. Ed. 2006, 45, 8160; J. E. Lee et al., J. Am. Chem. Soc. 2010, 132, 552].

However, to date, maximum $r_2$ relaxivity could not be realized since the fraction of magnetic nanoparticles in the clusters is relatively small. Therefore, the dispersible single core magnetic nanoparticles in SDR are highly desirable.

Korean Patent Application No. 10-2009-0125211 discloses an MRI contrast agent comprising iron oxide nanoparticles having a size of more than 40 nm. However, iron oxide nanoparticles having a size of more than 40 nm are

present in a solvent as aggregates having a size of more than 200 nm magnetic dipole interactions. When the aggregate is too large, the magnetic field of the aggregate is so strong and, thus, $T_2$ effects decreases. In addition, since nanoparticles are present as aggregates, as mentioned above, the retention time in bloodflow is too short. Moreover, the aggregates may cause damage of organs. Therefore, the conventional MRI contrast agent comprising iron oxide nanoparticles is only applicable to contrast cells.

The present inventors synthesized iron oxide nanoparticles having a size of 20 nm to 30 nm and, then, prepared dispersible ferrimagnetic iron oxide nanocomposite. The present inventors have completed the present invention by confirming that problems of the conventional MRI $T_2$ contrast agents can be overcome when the iron oxide nanocomposite of the present invention is used as MRI $T_2$ contrast agents.

## Disclosure

### Technical Problem

The primary object of the present invention is to provide an iron oxide nanocomposite comprising an iron oxide nanoparticle, wherein the iron oxide nanoparticle is encapsulated with a surfactant and the surfactant is encapsulated with polyethylene glycol-phospholipid.

Another object of the present invention is to provide an MRI (magnetic resonance imaging) $T_2$ contrast agent, comprising an iron oxide nanocomposite which includes an iron oxide nanoparticle, wherein the iron oxide nanoparticle is encapsulated with a surfactant and the surfactant is encapsulated with polyethylene glycol-phospholipid.

Yet another object of the present invention is to provide a method for preparing an iron oxide nanocomposite, comprising: (i) heating a mixture of an iron precursor, a surfactant and an organic solvent to form a ferromagnetic iron oxide nanoparticle encapsulated with a surfactant; and (ii) capping the ferromagnetic iron oxide nanoparticle encapsulated with a surfactant, with polyethylene glycol-phospholipid.

### Technical Solution

The primary object of present invention can be achieved by providing an iron oxide nanocomposite comprising an iron oxide nanoparticle, wherein the iron oxide nanoparticle is encapsulated with a surfactant and the surfactant is encapsulated with polyethylene glycol-phospholipid.

As used herein, the term "phospholipid" refers to an amphipathic molecule that includes a lipid region and hydrophilic region having phosphrus. Preferably the hydrophilic region may be phosphatidylserine, phosphatidylethanolamine, phosphatidylcholine or phosphatidylinositol.

As used herein, the term "polyethylene glycol (PEG)" refers to a polymeric compound that has a molecular weight of about 100 Da to about 10,000 Da according to the number of ethylene oxide in the polymer chain.

As used herein, the term "PEG-phospholipid" is a compound of the following chemical formula I.

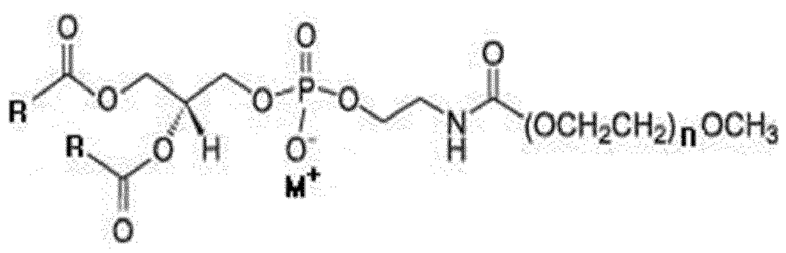

Formula I

In the chemical formula I, R is $C_{15}$-$C_{25}$ linear alkyl, $M^+$ is $NH^{4+}$ or $Na^+$, and n is an integer of 35 to 55.

The iron oxide nanocomposite of the present invention has a structure that a hydrophilic region of the surfactant chemically or physically binds to the surface of the ceria nanoparticle and two strands of alkyl (R) at the phospholipid region of the PEG-phospholipid encapsulate the lipophilic region of the surfactant.

In the iron oxide nanocomposite of the present invention, the iron oxide may be magnetite ($Fe_3O_4$) or maghemite (y- $Fe_3O_4$). Preferably, the iron oxide is magnetite.

The size of the iron oxide nanoparticle is preferably 20 nm to 30 nm. If the size of the iron oxide nanoparticle is more than 30 nm, $r_2$ relaxivity of the iron oxide nanoparticle decreases. The $r_2$ relaxivity of the iron oxide nanoparticle having a size of 20 nm to 30 nm is more than twice that of the iron oxide nanoparticle having a size of 40 nm. Moreover,

the iron oxide nanoparticle can migrate in vivo when the size of the iron oxide nanoparticle is less than or equal to 30 nm.

The surfactant which is included in the iron oxide nanocomposite of the present invention may be oleic acid, oleylamine, 4-biphenylcarboxylic acid, stearic acid or tri-n-octylphosphine oxide.

Another object of the present invention can be achieved by providing an MRI (magnetic resonance imaging) $T_2$ contrast agent, comprising an iron oxide nanocomposite which includes an iron oxide nanoparticle, wherein the iron oxide nanoparticle is encapsulated with a surfactant and the surfactant is encapsulated with polyethylene glycol-phospholipid

In the MRI $T_2$ contrast agent of the present invention, the iron oxide may be magnetite ($Fe_3O_4$) or maghemite (y- $Fe_3O_4$). Preferably, the iron oxide is magnetite.

The size of the iron oxide nanoparticle is preferably 20 nm to 30 nm. If the size of the iron oxide nanoparticle is more than 30 nm, $r_2$ relaxivity of the iron oxide nanoparticle decreases. The $r_2$ relaxivity of the iron oxide nanoparticle having a size of 20 nm to 30 nm is more than twice that of the iron oxide nanoparticle having a size of 40 nm. Moreover, the iron oxide nanoparticle can migrate in vivo when the size of the iron oxide nanoparticle is less than or equal to 30 nm.

The surfactant which is included in the MRI $T_2$ contrast agent of the present invention may be oleic acid, oleylamine, 4-biphenylcarboxylic acid, stearic acid or tri-n-octylphosphine oxide.

Yet another object of the present invention can be achieved by providing a method for preparing an iron oxide nanocomposite, comprising: (i) heating a mixture of an iron precursor, a surfactant and an organic solvent to form a ferromagnetic iron oxide nanoparticle encapsulated with a surfactant; and (ii) capping the ferromagnetic iron oxide nanoparticle encapsulated with a surfactant, with polyethylene glycol-phospholipid.

The iron precursor employed in the method for preparing an iron oxide nanocomposite of the present invention may be selected from iron acetylacetonate, iron chloride, iron acetate, iron sulfate or iron nitrate.

In addition, the surfactant may be oleic acid, oleylamine, 4-biphenylcarboxylic acid, stearic acid or tri-n-octylphosphine oxide.

Further, the organic solvent may be 4-biphenylcarboxylic acid, benzyl ether, phenyl ether, octyl ether, hexadecane, octadecene or mixtures thereof.

The heating temperature and time of the step (i) of the method for preparing an iron oxide nanocomposite of the present invention is preferably 200°C - 360°C and is 10 min - 3 hr, respectively.

The iron oxide synthesized in the step (i) of the method for preparing an iron oxide nanocomposite of the present invention may be magnetite or maghemite.

In the method for preparing an iron oxide nanocomposite of the present invention, a mole ratio of the iron precursor and the surfactant may be 1:0.1 - 1:20, and a mole ratio of the iron precursor and the organic solvent may be 1:1 - 1:1,000.

**Advantageous Effects**

The present invention may provide nanocomposites comprising iron oxide nanoparticles having a size of 20 nm to 30 nm, and the iron oxide nanocomposite has a use of a biocompatible MRI $T_2$ contrast agent.

In particular, since the $r_2$ relaxivity of the MRI $T_2$ contrast agent of the present invention is more than twice that of the conventional MRI $T_2$ contrast agent compriaing iron oxide nanoparticles having a size of more than 30 nm, it is possible to obtain significant $T_2$ contrasting effects.

**Brief Description of the Drawings**

Fig. 1a is TEM images of 22-nm iron oxide nanocomposites dispersed in water, in Example 2 of the present invention, Fig. 1b is an image showing high colloidal stability of iron oxide nanocomposites in water, Fig. 1c is DLS data for iron oxide nanocomposites in water, and Fig. 1d is M-H curves of ferrimagnetic iron oxide nanoparticles.

Fig. 2a and 2b are DLS data of 22-nm iron oxide nanocomposites encapsulated with PEG-phospholipid in PBS(Fig. 2a) and culture media containing 10% FBS (Fig. 2b), Fig. 2c is DLS data of FION (ferrimagnetic iron oxide nanoparticle) dispersed in chloroform, and Fig. 2d is DLS data of 22-nm FION encapsulated with CTAB in water.

Fig. 3 is the relaxivity of iron oxide nanocomposites in water and in agarose of the present invention.

Fig. 4 shows MR contrast effect of ferrimagnetic iron oxide nanoparticles on changes in size. Fig. 4a is $T_2$-weighted MR images of ferrimagnetic iron oxide nanoparticles at various concentrations of iron at 3 T, Fig. 4b is color-coded images of Fig. 4a, Fig. 4c is plots of $r_2$ values of the ferrimagnetic iron oxide nanoparticles, and Fig. 4d is plots of

$r_2$ values in accordance with size of the ferrimagnetic iron oxide nanoparticles.

Fig. 5 shows TEM images and DLS data of 28-nm (Fig. 5a), 32-nm (Fig. 5b), 42-nm (Fig. 5c), and 49-nm (Fig. 5d) ferrimagnetic iron oxide nanoparticles.

Fig. 6a is *in vitro* cytotoxicity test of iron oxide nanocomposites of the present invention, and Fig. 6b is $T_2$-weighted MR image of dispersed cells in agarose.

Fig. 7 is confocal laser scanning microscopic images of B16F10 cells incubated with iron oxide nanocomposites for 24 h.

Fig. 8 is *in vivo* MR Images of the tumor site before (Fig. 8a) and 1h after (Fig. 8b) intravenous injection of iron oxide nanocomposites (arrows indicate the tumor sites).

Fig. 9 is fluorescence images of sectioned tissues stained with DAPI (4',6-diamidino-2-phenylindole). The iron oxide nanocomposites of the present invention (red for RITC) were detected at spleen, liver, and tumor.

Fig. 10 is *in vivo* MR images of lymph nodes (arrows) in nude mouse before and 1 h after intravenous administration of the iron oxide nanocomposites of the present invention.

Fig. 11 is images of H&E stained sections of mouse organs after intravenous administration of the iron oxide nanocomposites of the present invention.

**Best Mode for Carrying Out the Invention**

Hereinafter, the present invention will be described in greater detail with reference to the following examples and drawings. The examples and drawings are given only for illustration of the present invention and not to be limiting the present invention.

**Example 1. Preparation of iron oxide nanocomposite**

Iron (III) acetylacetonate (0.706 g, 2 mmol, Acros, 99%) was added to a mixture composed of oleic acid (1.27 g, 4 mmol, Aldrich, 90%), 4-biphenylcarboxylic acid (0.4 g, Acros 95%), and benzyl ether (10.40 g, 10 ml, Aldrich, 99%,). The mixture solution was degassed at room temperature for 1 hour. The solution was then heated to 290°C at the heating rate of 20°C/min with vigorous magnetic stirring to prevent aggregation. The reaction mixture was maintained at this temperature for 30 minutes. After cooling the solution to room temperature, ethanol or acetone was added to the solution. The solution was then centrifuged at 1,700 rpm for 10 minutes to precipitate the particles. The separated precipitate (ferrimagnetic iron oxide nanoparticle (FION)) was dispersed in nonpolar solvent such as chloroform and n-hexane (10 ml).

The resulting nanoparticles were then encapsulated by PEG-phospholipid shell to endow them with biocompatibility and water-dispersibility. Typically, 2 ml of the organic dispersible FIONs in $CHCl_3$ (5 mg/ml) was mixed with 1 ml of $CHCl_3$ containing 10 mg of 1, 2-distearoyl-sn-glycero-3-phosphoethanolamine-N-[methoxy(polyethylene glycol)-2000] (PEG-phospholipid, Avanti Polar Lipids, Inc.). After evaporating the solvent, the resulting mixture was incubated at 80°C in vacuum for 1 hr. Subsequently 2 ml of sterilized water was added and sonicated to disperse the nanoparticles. Excess PEG-phospholipid was removed by ultracentrifugation at 20,000 rpm for 1 hr. After ultracentrifugation, the iron oxide nanocomposite dispersion was filtered through a cellulose acetate syringe filter (Advantec, Japan) to remove any aggregates or contaminants.

**Example 2. Characterization of iron oxide nanocomposite**

Transmission electron microscopy (TEM) images of the iron oxide nanocomposite obtained in Example 1 were taken on a JEOL JEM-2010 electron microscope at 200 kV. Samples were prepared by dropping small volume of particle dispersion onto a carbon-coated copper grid. The hydrodynamic diameters of nanoparticles were measured with a particle size analyzer (ELS-Z2, Otsuka). M-H curves were obtained by the vibrating sample magnetometer (VSM, Quantum Design PPMS). The iron concentrations of nanoparticles were measured with inductively coupled plasma atomic emission spectroscopy (ICP-AES) using ICPS-7500 spectrometer (Shimadzu).

The transmission electron microscopy (TEM) image shows that cube-shaped nanoparticles were obtained with an average size of $22 \pm 2.6$ nm (Fig. 1a). Since the nanoparticles synthesized in the present Exmaple are very hydrophobic, the iron oxide nanocomposites were transferred to aqueous media by encapsulation with polyethylene glycol-phospholipid (PEG-phospholipid). The encapsulated iron oxide nanocomposites showed excellent colloidal stability in water.

Furthermore, the iron oxide nanocomposites did not aggregate even in an external magnetic field (Fig. 1b), and the hydrodynamic diameter in deionized water, measured with dynamic light scattering (DLS), was 43±10 nm (Fig. 1c). In addition, the hydrodynamic diameters of the iron oxide nanocomposites were 51±6.5 nm and 52±10.9 nm in phosphate buffered saline (PBS) and culture media containing 10% fetal bovine serum (FBS), respectively, demonstrating that they are colloidally stable in biological media (Figs. 2a and 2b). As shown in Fig. 1d, the saturation magnetization of the 22-nm iron oxide nanocomposites was very similar to that of the larger-sized nanocubes since the portion of canted spins on the surface of the nanoparticles is very small in this size range.

However, coercivity and remnant magnetization of single-domain magnetic nanoparticles decreased with decreasing size (Fig. 1d). The remnant magnetization and coercivity of the 22-nm iron oxide nanocomposites are ca. 5 emu $g^{-1}$(Fe) and ca. 14 Oe, respectively, at room temperature.

Recently, colloidally stable ferromagnetic $Fe/Fe_3O_4$ nanoparticles with relatively small coercivity and remnant magnetization were reported, demonstrating that the magnetic dipole interaction between nanoparticles can be overcome using suitable surfactants. Compared to these core/shell $Fe/Fe_3O_4$ nanoparticles, the coercivity and remnant magnetization of the iron oxide nanocomposites are smaller, whereas the core size is larger. In addition to the small remnant magnetization, the stabilization by long PEG chains is critical for achieving colloidal stability in aqueous media. The measured hydrodynamic diameter of the as-synthesized ferrimagnetic iron oxide nanoparticles in chloroform was 99±24 nm (Fig. 2c), and these nanoparticles were aggregated in the presence of an external magnetic field. When the nanoparticles were dispersed in water using charge repulsion by encapsulation with cetyltrimethylammonium bromide (CTAB), the hydrodynamic diameter was 96±24 nm (Fig. 2d), which is still larger than that of the iron oxide nanocomposites encapsulated with PEG-phospholipids.

**Example 3. Measuring of MR relaxivity of ferrimagnetic iron oxide nanoparticles (FIONs)**

In order to measure MR relaxivity, ferrimagnetic iron oxide nanoparticles, obtained in Example 1, of 22, 28, 32, 42, and 49 nm in size were dispersed in 1% agarose solution (analytical grade agarose; Promega) to prevent sedimentation. $T_2$ values of the nanoparticles were measured using the Carr-Purcell-Meiboom-Gill (CPMG) sequence with a head coil on a 3-T MR scanner (TrioTrim, Siemens): TR = 5000 ms, TE = 16, 32, 48, 64, 20, 40, 60, 80, 50, 100, 150, 200 ms. Fast spin echo $T_2$-weghted MR images of the phantom were acquired using the following parameters: flip angle = 120, ETL = 18, TR = 6000 ms, TE = 90 ms, field of view FOV = 119 × 170 $mm^2$, matrix = 448 × 640, slice thickness / gap = 1.4 mm / 1.8 mm, NEX = 1.

The $r_2$ relaxivities of the iron oxide nanocomposites and larger ferrimagnetic iron oxide nanoparticles were measured using a 3-T clinical MR scanner. Given that the ferrimagnetic iron oxide nanoparticles larger than 30 nm are not colloidally stable, these nanoparticles were dispersed in 1% agarose to prevent sedimentation during the measurement. Dispersion in agarose did not affect the $T_2$ relaxation process, and the $r_2$ values of the iron oxide nanocomposites dispersed in water and agarose were nearly identical (Fig. 3). The $T_2$-weighted MR images show that 22 nm-sized iron oxide nanocomposites produced stronger $T_2$ contrast effect than the larger nanocubes (Figs. 4a and 4b). The $r_2$ values of the iron oxide nanoparticles with sizes of 22, 28, 32, 42, and 49 nm were 761, 740, 532, 343, and 296 $s^{-1}mM^{-1}$, respectively (Figs. 4c, 4d).

In general, the $r_2$ values of magnetic nanoparticles increase with increasing size. However, as shown in Fig. 4d, the $r_2$ values of the 22-nm- and 28-nm-sized iron oxide nanocomposites are very similar, and the $r_2$ values decreased for the nanoparticles larger than 30 nm. According to theoretical studies, the highest $r_2$ relaxivity is expected to be achieved for spherical single-core iron oxide nanoparticles with diameters ranging from 30 nm to 300 nm, and multicore iron-oxide-nanoparticle clusters ranging in size from 30 nm to 120 nm. The core volume of cube-shaped iron oxide nanocomposites with an edge dimension of 22 nm is similar to that of spherical nanoparticles with a diameter of 30 nm. The hydrodynamic diameters of the 22-nm- and 28-nm-sized ferrimagnetic nanoparticles are 44 nm and 56 nm, respectively, demonstrating that the iron oxide nanocomposites are in the static dephasing regime (SDR) where the highest $r_2$ value is expected to appear. In the SDR, the $r_2$ value of the nanoparticle is given by

$$r_2 = \frac{8\pi^2\sqrt{3}}{81}\frac{A^3 N_0}{10^6 Z}\gamma M_s \qquad \text{Equation (1)}$$

where A is the lattice parameter, $N_0$ is the Avogadro constant, Z is the number of formula units per unit cell, $\gamma$ is the gyromagnetic ratio, and $M_s$ is the saturation magnetization. As shown in equation (1), the $r_2$ value in the SDR is dependent solely on the saturation magnetization. The $r_2$ value of the 22-nm-sized ferrimagnetic iron oxide nanoparticle with a

saturation magnetization of 106 emu $g^{-1}$(Fe) is calculated to be approximately 800 $s^{-1}mM^{-1}$, which is very similar to the measured $r_2$ value of the 22-nm iron oxide nanocomposites (761 $s^{-1}mM^{-1}$).

Iron oxide nanoparticles larger than 30 nm aggregate because of their strong magnetic dipole moments, and the overall size becomes larger than 200 nm. The hydrodynamic diameters of 32-, 42-, and 49-nm-sized ferrimagnetic iron oxide nanoparticles are 261, 378, and 534 nm, respectively, which are beyond the SDR (Fig. 5). Since these large aggregates of magnetic nanoparticles generate a very strong magnetic field, the nearby water protons are completely dephased and they are unable to contribute to the MR signal. Consequently, for these large aggregates, the $r_2$ relaxivity decreases with increasing nanoparticle size (referred to as the "Luz-Meiboom" regime).

**Example 4. Cytotoxicity and cellular uptake of FION**

B16F10 melanoma cells were grown in Dulbecco's Modified Eagle's Medium (DMEM, Welgene) supplemented with 10% (v/v) fetal bovine serum (FBS, Gibco) and penicillin/streptomycin (100 U/mL and 100 μg/mL, respectively, Gibco) at 37°C in humidified 5% $CO_2$ atmosphere.

The viability of the cells in the presence of iron oxide nanocomposites was evaluated using 3-[4,5-dimethylthialzol-2-yl]-2,5-diphenyltetrazolium bromide (MTT, Sigma) assay. The assay was performed in triplicate in the following manner. For MTT assay, the cells were seeded into 96-well plates at a density of $1 \times 10^4$ per well in 200 L of media and grown overnight. The cells were then incubated at various concentrations of FION (0, 0.012, 0.023, 0.047, 0.094, 0.19, 0.38, 0.75 mg (Fe) $mL^{-1}$) for 24 h. Following this incubation, the cells were incubated in media with 0.1 mg $ML^{-1}$ of MTT for 1 h. Then the MTT solution was removed and the precipitated violet crystals were dissolved in 200 L of DMSO. The absorbance was measured at 560 nm with Sunrise™ microplate reader (Tecan Trading AG). No appreciable toxicity was observed up to a very high concentration of 0.75 mg(Fe) $mL^{-1}$, demonstrating the high biocompatibility of iron oxide nanocomposites (Fig. 6a).

To observe cellular uptake of nanoparticles, the cells were cultured in a 4-well chamber slide (Nalgen Nunc, Naperville, IL) and incubated with iron oxide nanocomposites. After 24 h, the cells were washed with phosphate buffered saline (PBS), fixed with 4% paraformaldehyde, and stained with 4',6-diamidino-2-phenylindole (DAPI, 1 μg/mL in PBS, Roche). The fluorescence images were acquired with confocal laser scanning microscopy (LSM 510, Carl Zeiss, Germany). The iron oxide nanocomposites were readily internalized by the cells without any treatment to enhance cellular uptake and they were observed in the cytoplasm as red fluorescence (Fig. 7).

**Example 5. *In vitro* MR imaging**

In order to label the cells with iron oxide nanoparticles, the cells were seeded onto culture dishes in 10 mL of media and grown overnight. Subsequently, iron oxide nanocomposites of 0, 3.13, 6.25, 12.5, 25, and 50 μg/mL were added. After 24 h, the cells were washed twice with PBS and detached by adding 1 mL of trypsin/EDTA (Gibco). After centrifugation, cells were suspended in 1% agarose. $T_2$-weighted MR images were acquired with a head coil on a 3-T MR scanner. The MR signal intensities of the cells were clearly attenuated as the concentration of iron oxide nanocomposites increased (Fig. 6b).

**Example 6. *In vivo* MR imaging**

B16F10 cells ($5 \times 10^5$) in 50 μL of serum-free media were mixed with an equivalent volume of matrigel (BD Biosciences) at 4 °C, followed by subcutaneous injection into the flank of nude mouse. After 3 weeks, in vivo gradient $T_2$*-MR images of the nude mouse were acquired using a home-made small animal coil with 6 channel on a 3 T MRI scanner before and after the injection of iron oxide nanocomposites (10 mg (Fe) $kg^{-1}$) into the tail vein. The measurement parameters are as follows: flip angle = 12, ETL = 1, TR = 40 ms, TE = 22 ms, field of view FOV = 70 $\times$ 49 mm, matrix = 256 $\times$ 180, slice thickness / gap = 0.6 mm / 0 mm.

In the MR images of Fig. 8, signal attenuation at the tumor site was clearly observed 1 h after injecting the iron oxide nanocomposites, demonstrating the passive targeting of iron oxide nanocomposites.

In order to confirm the accumulation of iron oxide nanocomposites at the tumor site, the mouse was sacrificed after the MR imaging, and sections of the tumor were observed with a fluorescence microscope (Fig. 9). When the tissue sections were examined using fluorescence microscope, a very small amount of iron oxide nanocomposites was observed in the lung, indicating that large agglomerates did not form during the circulation (Fig. 9). The fluorescence image shows the accumulation of RITC-labeled iron oxide nanocomposites throughout the tumor site. In addition to the tumor, signal attenuation can be observed in the liver, spleen, bone marrow, and lymph nodes owing to the clearance of the nanoparticles by macrophages (Fig. 10). The signal decrease in the lymph nodes demonstrates that iron oxide nanocomposites can also be used for the detection of lymph node metastases.

**Example 7. Histochemical analysis**

After MRI scans, iron oxide nanocomposites injected mice were sacrificed under anesthetic conditions and tissues of interest (tumor, kidney, liver, spleen, lung, and heart) were excised and fixed in 10% neutral buffered (10% NBF) for 1 week. For haematoxylin and eosin (H&E) staining, formalin fixed tissues from each organ were embedded into paraffin and paraffin-embedded tissues were sectioned into 4 μm thickness. Tissues samples were dewaxed, hydrated and standard H&E staining was performed to evaluate morphological features of each organs. Stained images were acquired with optical microscope (BX53P, Olympus, Japan). For fluorescence staining, formalin fixed tissues were frozen with liquid nitrogen and cryosectioned into 10 μm thickness. Samples were immersed with 4'-6-diamidino-2-phenylindole (DAPI) for 5 minutes at room temperature to visualize the nucleus of cells and images were acquired with fluorescence microscope (Leica DM2500, Leica, Germany) (Fig. 11). As shown in Fig. 11, no evidence of any adverse effect due to iron oxide nanocomposites was observed.

## Claims

1. An iron oxide nanocomposite comprising an iron oxide nanoparticle, wherein said iron oxide nanoparticle is encapsulated with a surfactant and said surfactant is encapsulated with polyethylene glycol-phospholipid.

2. The iron oxide nanocomposite of Claim 1, wherein said iron oxide is magnetite or maghemite.

3. The iron oxide nanocomposite of Claim 1, wherein a size of said iron oxide nanoparticle is 20 nm - 30 nm.

4. The iron oxide nanocomposite of Claim 1, wherein said surfactant is selected from the group consisting of oleic acid, oleylamine, 4-biphenylcarboxylic acid, stearic acid and tri-n-octylphosphine oxide.

5. An MRI (magnetic resonance imaging) $T_2$ contrast agent, comprising an iron oxide nanocomposite which includes an iron oxide nanoparticle, wherein said iron oxide nanoparticle is encapsulated with a surfactant and said surfactant is encapsulated with polyethylene glycol-phospholipid.

6. The MRI contrast agent of Claim 5, wherein said iron oxide is magnetite or maghemite.

7. The MRI contrast agent of Claim 5, wherein a size of said iron oxide nanoparticle is 20 nm - 30 nm.

8. The MRI contrast agent of Claim 5, wherein said surfactant is selected from the group consisting of oleic acid, oleylamine, 4-biphenylcarboxylic acid, stearic acid and tri-n-octylphosphine oxide.

9. A method for preparing an iron oxide nanocomposite, comprising:

   (i) heating a mixture of an iron precursor, a surfactant and an organic solvent to form a ferromagnetic iron oxide nanoparticle encapsulated with a surfactant; and
   (ii) capping said ferromagnetic iron oxide nanoparticle encapsulated with a surfactant, with polyethylene glycol-phospholipid.

10. The method of Claim 9, wherein said iron precursor is selected from the group consisting of iron acetylacetonate, iron chloride, iron acetate, iron sulfate and iron nitrate.

11. The method of Claim 9, wherein said surfactant is selected from the group consisting of oleic acid, oleylamine, 4-biphenylcarboxylic acid, stearic acid and tri-n-octylphosphine oxide.

12. The method of Claim 9, wherein said organic solvent is at least one selected from the group consisting of 4-biphenylcarboxylic acid, benzyl ether, phenyl ether, octyl ether, hexadecane and octadecene.

13. The method of Claim 9, wherein a heating temperature of the step (i) is 200°C - 360°C, a heating time is 10 min - 3 hr.

14. The method of Claim 9, wherein said iron oxide is magnetite or maghemite.

15. The method of Claim 9, wherein a mole ratio of said iron precursor and said surfactant is 1:0.1 - 1:20.

**16.** The method of Claim 9, wherein a mole ratio of said iron precursor and said organic solvent is 1:1 - 1:1,000.

Fig. 1

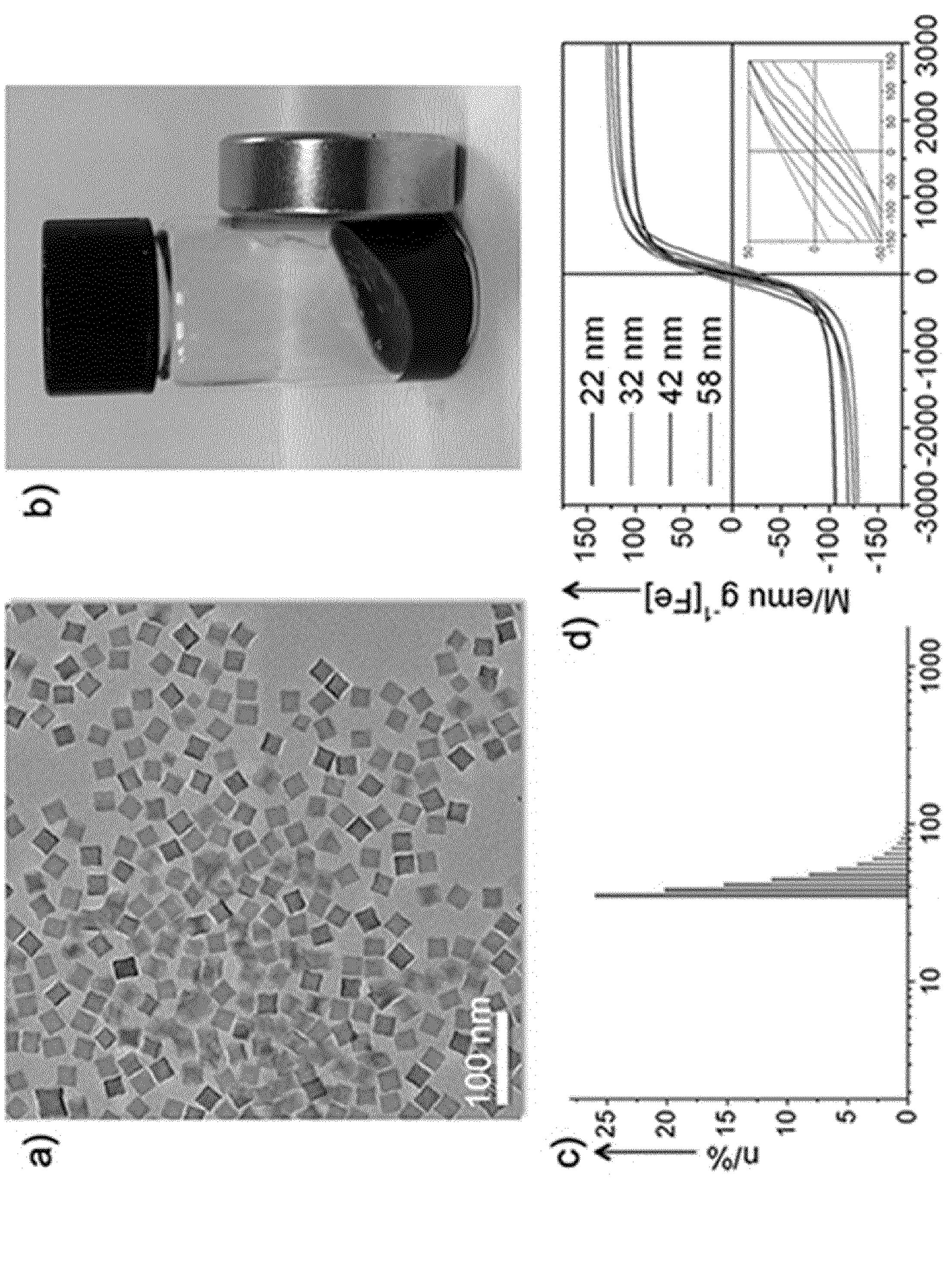

Fig. 2

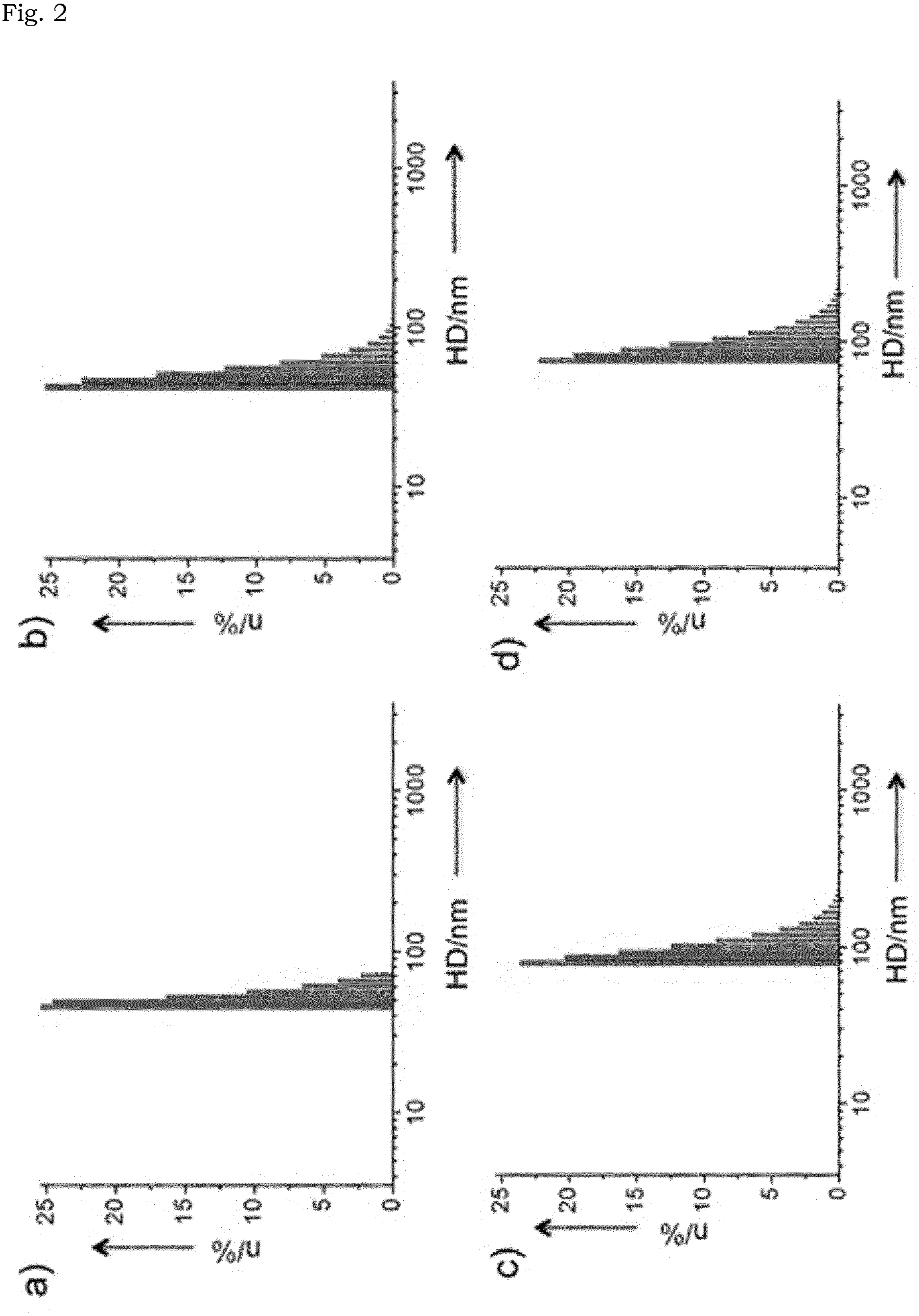

Fig. 3

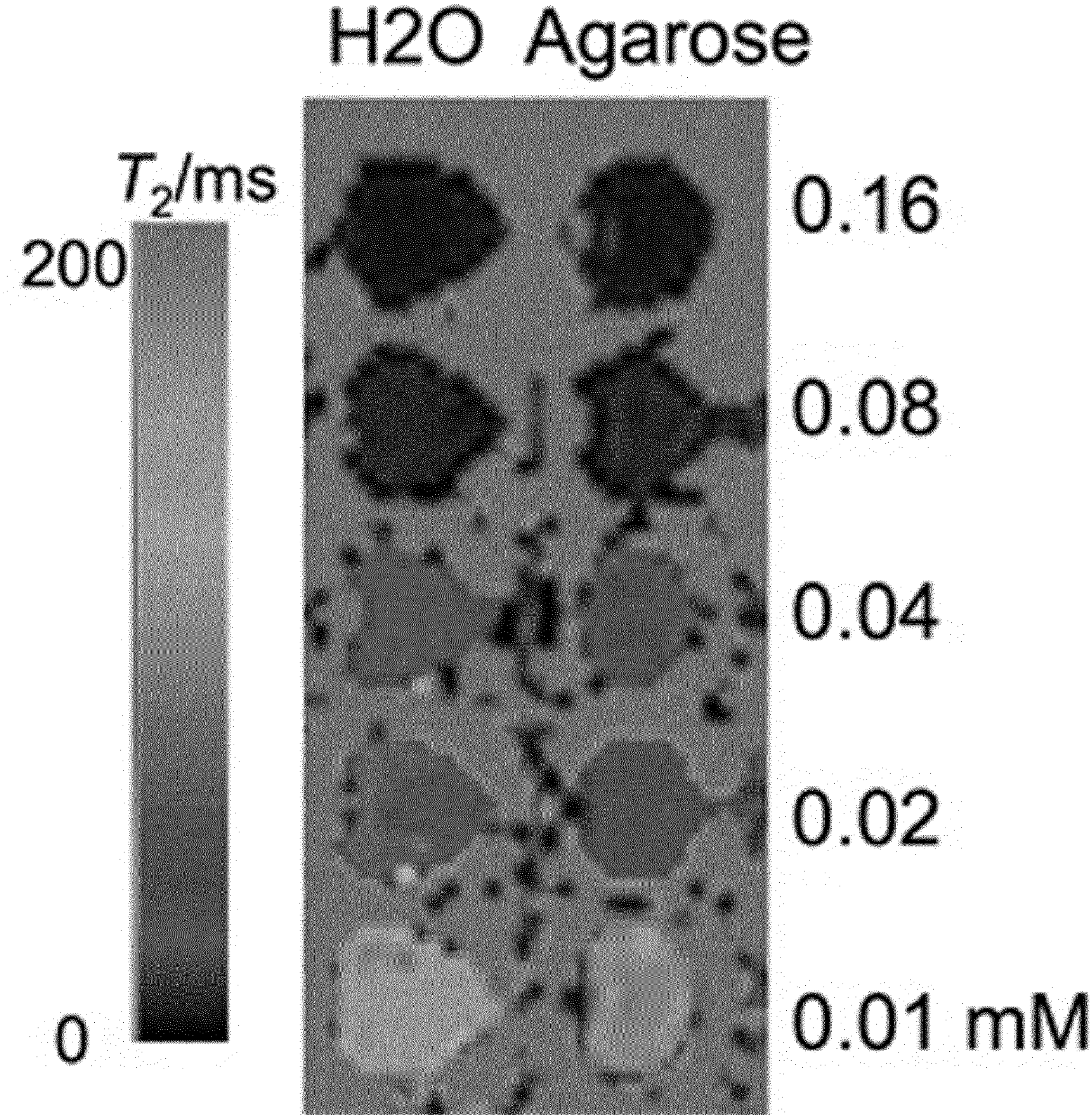

Fig. 4

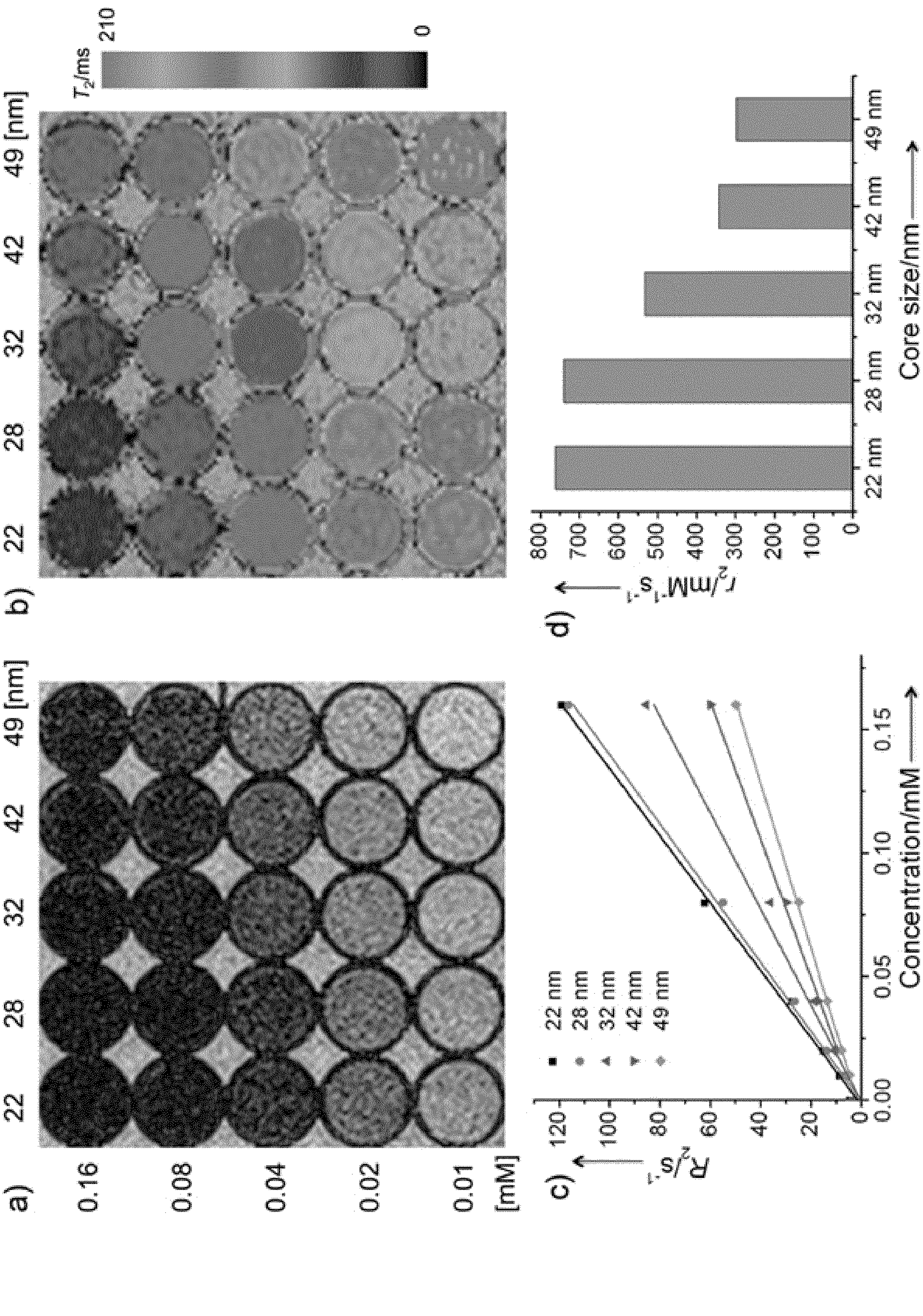
a)
0.16
0.08
0.04
0.02
0.01 [mM]
22
28
32
42
49 [nm]
b)
$T_2$/ms
210
0
c)
$R_2$/s$^{-1}$
Concentration/mM
22 nm
28 nm
32 nm
42 nm
49 nm
d)
$r_2$/mM$^{-1}$s$^{-1}$
Core size/nm

Fig. 5

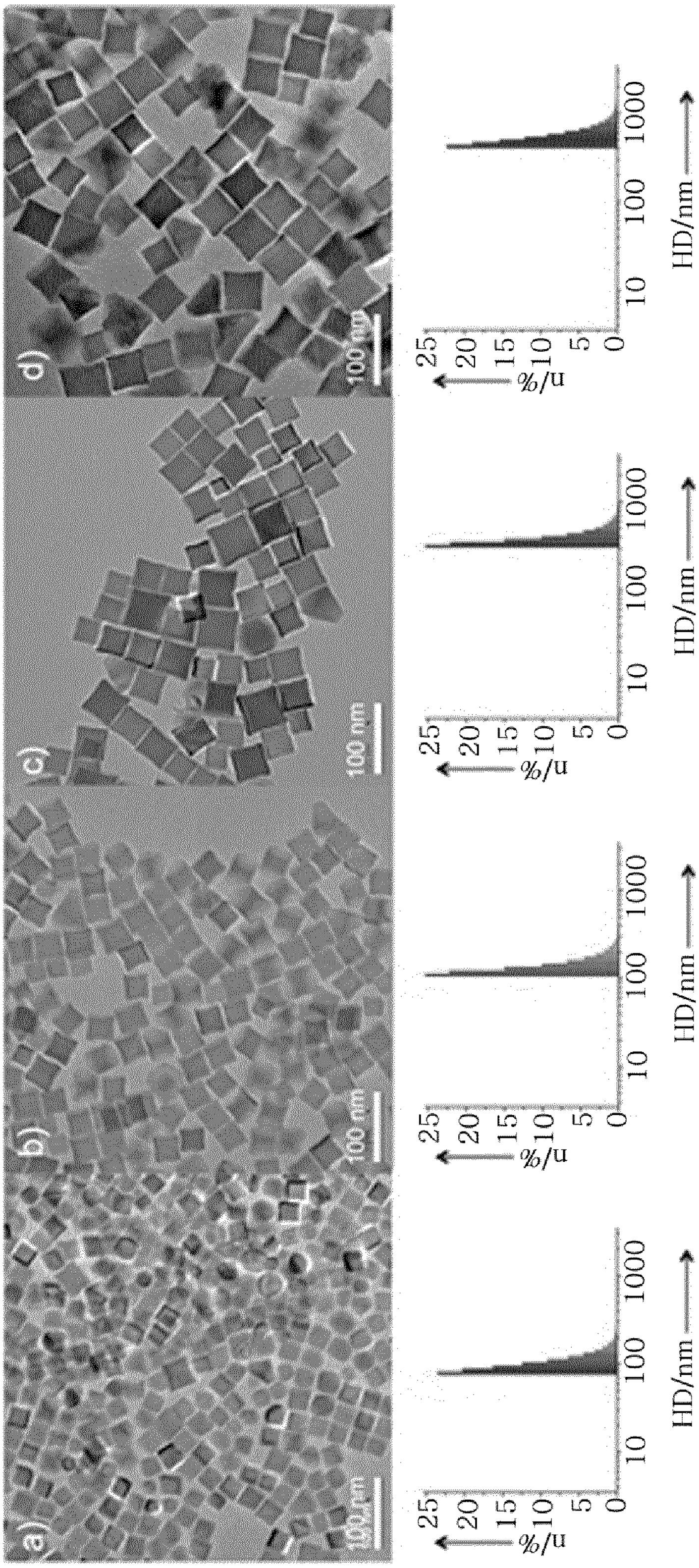

Fig. 6

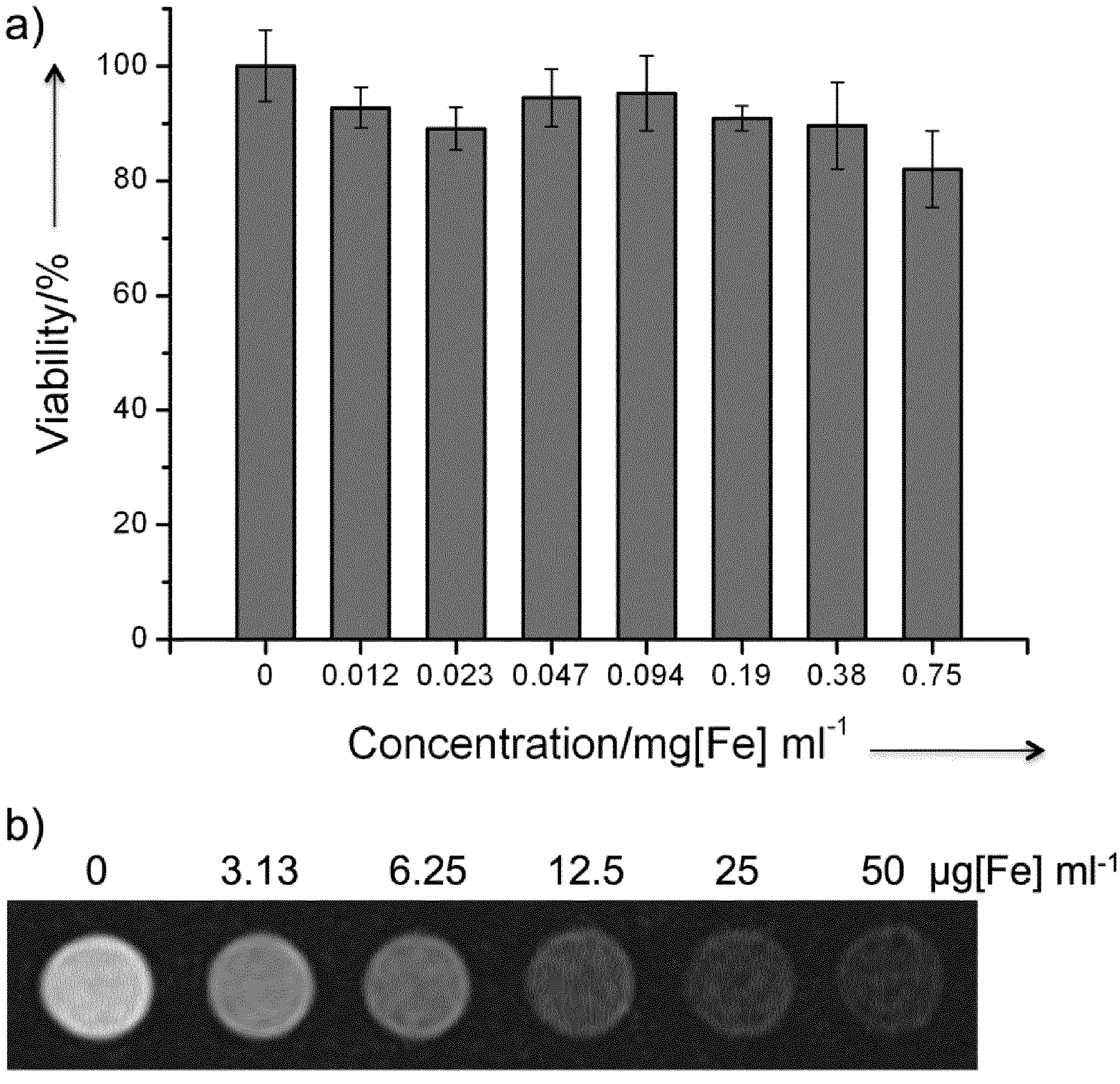
a)
Viability/%
0
20
40
60
80
100
0
0.012
0.023
0.047
0.094
0.19
0.38
0.75
Concentration/mg[Fe] ml-1
b)
0
3.13
6.25
12.5
25
50
µg[Fe] ml-1

Fig. 7

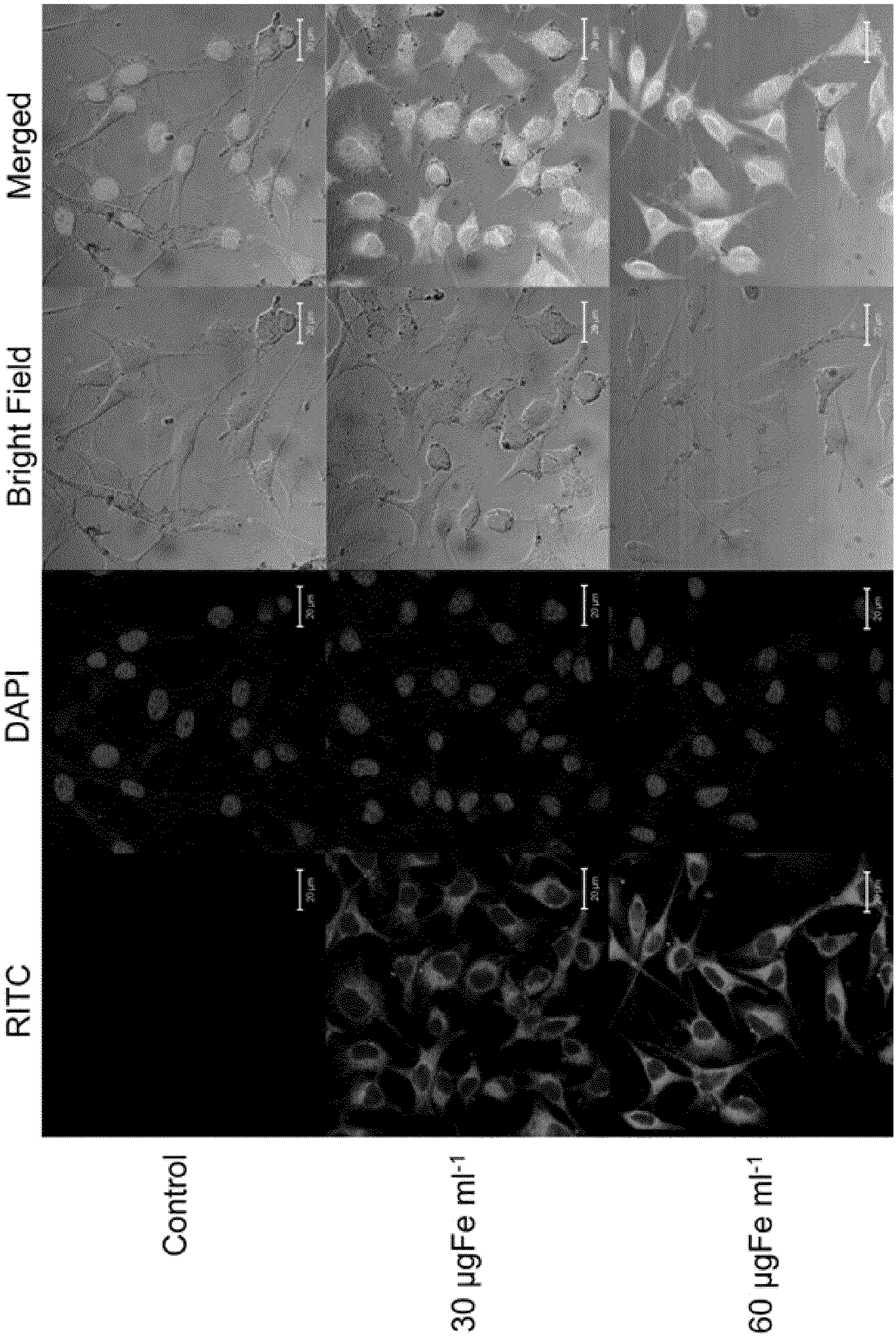
Merged
Bright Field
DAPI
RITC
Control
30 μgFe ml-1
60 μgFe ml-1

Fig. 8

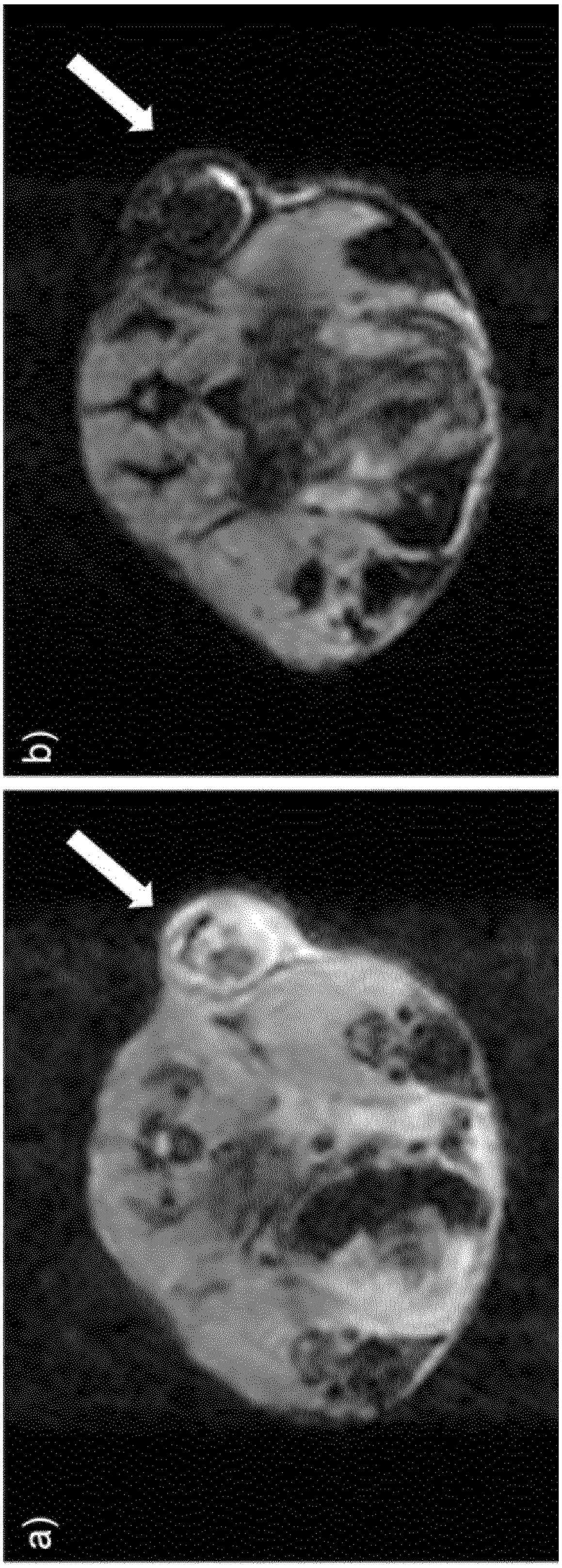

Fig. 9

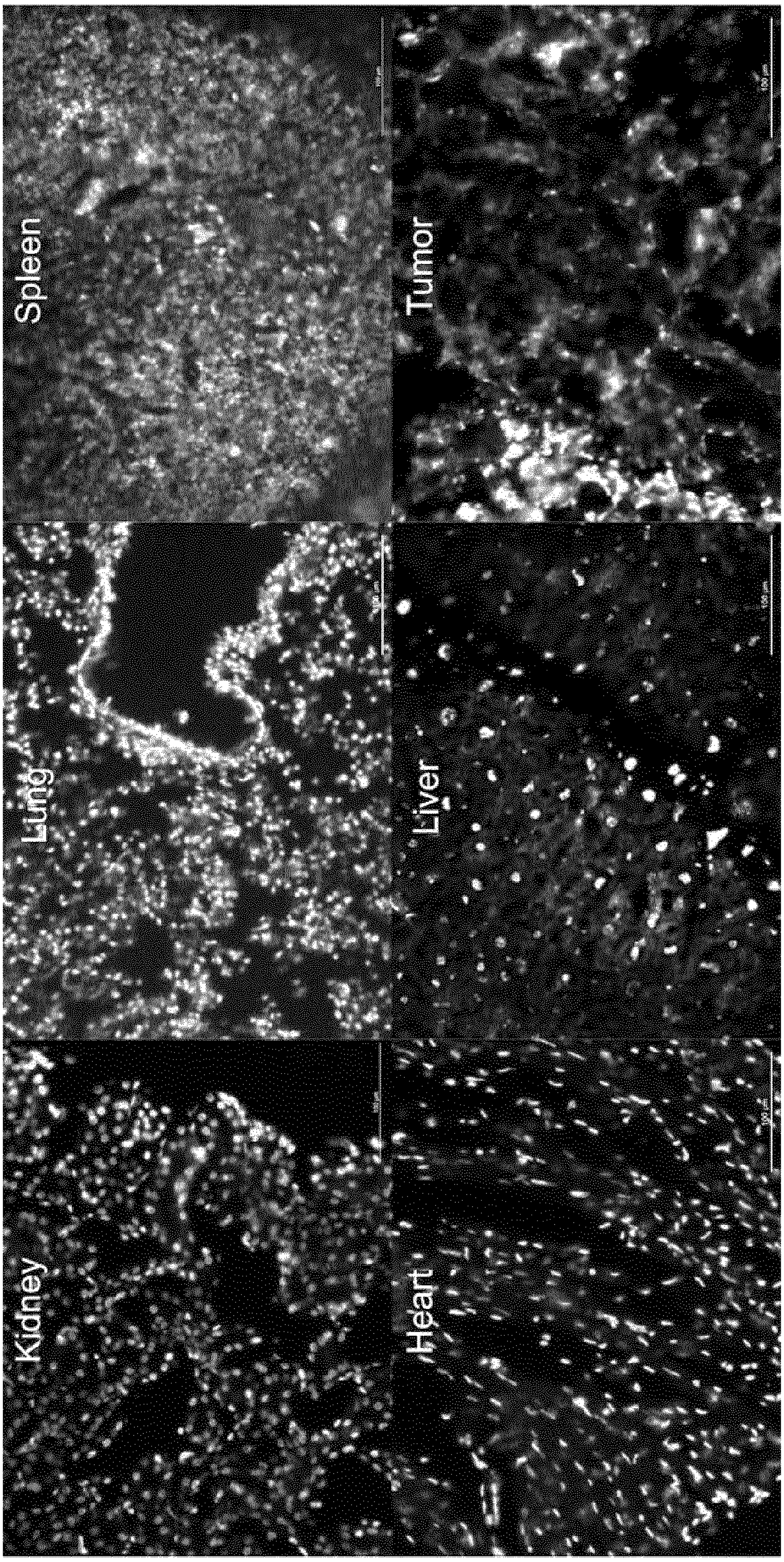

Fig. 10

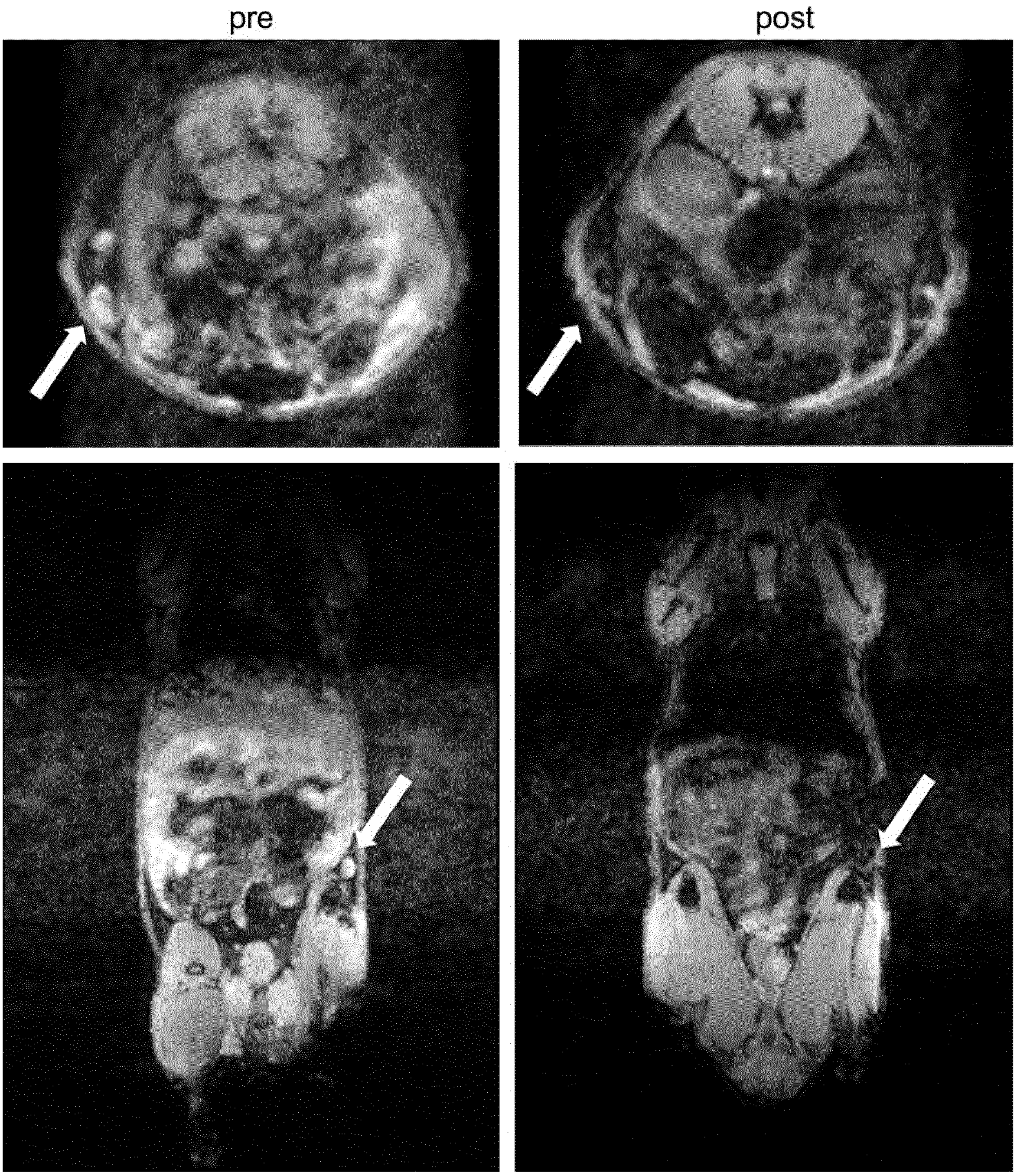

Fig. 11

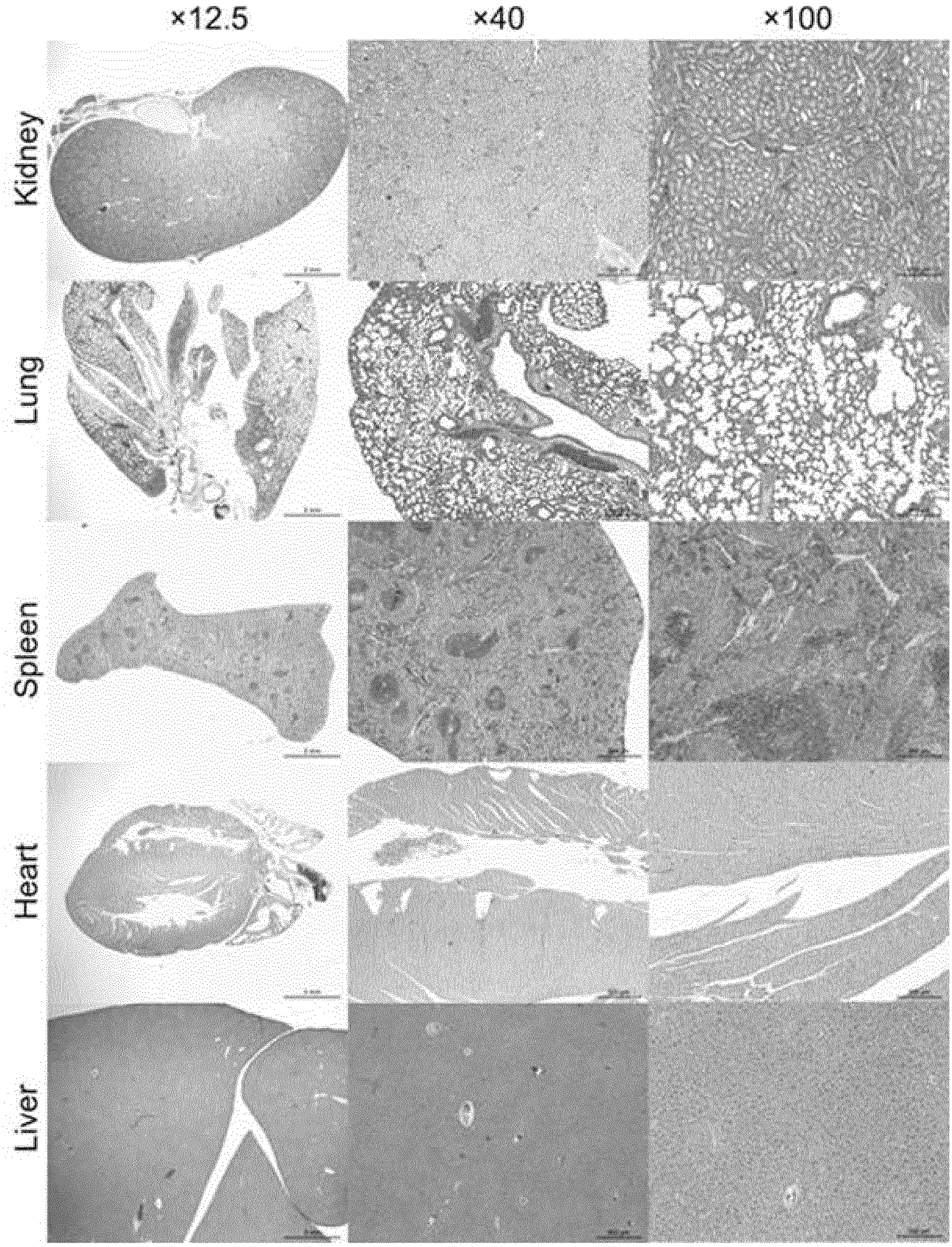

## INTERNATIONAL SEARCH REPORT

International application No.
**PCT/KR2013/002666**

A. CLASSIFICATION OF SUBJECT MATTER

***A61K 49/06(2006.01)i, A61K 9/16(2006.01)i, A61K 9/14(2006.01)i***

According to International Patent Classification (IPC) or to both national classification and IPC

B. FIELDS SEARCHED

Minimum documentation searched (classification system followed by classification symbols)
A61K 49/06; A61K 49/08; A61K 49/18; A61K 9/16; A61K 9/14

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: ferri-magnetism, surfactant, oxidized steel, polyethylene glycol, phospholipid, magnetite, T2 contrast media

C. DOCUMENTS CONSIDERED TO BE RELEVANT

| Category* | Citation of document, with indication, where appropriate, of the relevant passages | Relevant to claim No. |
|---|---|---|
| X | LEE, N. et al., "Magnetosome-like ferrimagnetic iron oxide nanocubes for highly sesitive MRI of single cells and transplanted pancreatic islets" PNAS, 2011, vol. 108, no. 7, pages 2662-2667. See abstract, pages 2662 and 2663. | 1,2,4-6,8-12,14-16 |
| Y | | 13 |
| A | | 3,7 |
| Y | KIM, D. et al., "Synthesis of uniform ferrimagnetic magnetite nanocubes" Journal of the American Chemical Society, 2009, vol. 131, pages 454-455. See abstract and page 454. | 13 |
| A | KR 10-2011-0068323 A (SNU R&DB FOUNDATION) 22 June 2011 See abstract and claims 1-5, 11, 12 and 18-23. | 1-16 |
| A | TONG, S. et al., "Coating optimization of superparamagnetic iron oxide nanoparticles for high T2 relaxivity", Nano Letters, 2010, vol. 10, pages 4607-4613. See abstract, pages 4607, 4608 and figure 1. | 1-16 |
| A | KR 10-2011-0080781 A (KOREA BASIC SCIENCE INSTITUTE) 13 July 2011 See abstract, paragraphs [0001], [0016], [0022] and [0023], claims 1, 3 and figure 1. | 1-16 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 AUGUST 2013 (09.08.2013) | **12 AUGUST 2013 (12.08.2013)** |
| Name and mailing address of the ISA/KR<br>Korean Intellectual Property Office<br>Government Complex-Daejeon, 189 Seonsa-ro, Daejeon 302-701, Republic of Korea<br>Facsimile No. 82-42-472-7140 | Authorized officer<br><br>Telephone No. |

Form PCT/ISA/210 (second sheet) (July 2009)

INTERNATIONAL SEARCH REPORT
Information on patent family members

International application No.
**PCT/KR2013/002666**

| Patent document cited in search report | Publication date | Patent family member | Publication date |
|---|---|---|---|
| KR 10-2011-0068323 A | 22/06/2011 | NONE | |
| KR 10-2011-0080781 A | 13/07/2011 | KR 10-1126726 B1<br>US 2011-0165086 A1 | 29/03/2012<br>07/07/2011 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020090125211

### Non-patent literature cited in the description


- **Y.-W. JUN ; J.-H. LEE ; J. CHEON.** *Angew. Chem.,* 2008, vol. 120, 5200
- *Angew. Chem. Int. Ed.,* 2008, vol. 47, 5122
- **H. B. NA ; I. C. SONG ; T. HYEON.** *Adv. Mater.,* 2009, vol. 21, 2133
- **J. XIE ; G. LIU ; H. S. EDEN ; H. AI ; X. CHEN.** *Acc. Chem. Res.,* 2011, vol. 44, 883
- **M. SRINIVAS ; E. H. J. G. AARNTZEN ; J. W. M. BULTE ; W. J. OYEN ; A. HEERSCHAP ; I. J. M. DE VRIES ; C. G. FIGDOR.** *Adv. Drug. Deliv. Rev.,* 2010, vol. 62, 1080
- **N. LEE ; T. HYEON.** *Chem. Soc. Rev.,* 2012
- **P. BOURRINET ; H. H. BENGELE ; B. BONNEMAIN ; A. DENCAUSSE ; J.-M. IDEE ; P. M. JACOBS ; J. M. LEWIS.** *Invest. Radiol.,* 2006, vol. 41, 313
- **T. K. JAIN ; M. K. REDDY ; M. A. MORALES ; D. L. LESLIE-PELECKY ; V. LABHASETWAR.** *Mol. Pharm.,* 2008, vol. 5, 316
- **Y.-W. JUN ; Y.-M. HUH ; J.-S. CHOI ; J.-H. LEE ; H.-T. SONG ; S. KIM ; S. YOON ; K.-S. KIM ; J.-S. SHIN ; J.-S. SUH.** *J. Am. Chem. Soc.,* 2005, vol. 127, 5732
- **R. A. BROOKS ; F. MOINY ; P. GILLIS.** *Magn. Reson. Med.,* 2001, vol. 45, 1014
- **R. A. BROOKS.** *Magn. Reson. Med.,* 2002, vol. 47, 388
- **D. KIM ; N. LEE ; M. PARK ; B. H. KIM ; K. AN ; T. HYEON.** *J. Am. Chem. Soc.,* 2009, vol. 131, 454
- **N. LEE et al.** *Proc. Natl. Acad. Sci. USA,* 2011, vol. 108, 2662
- **P. MOROZ ; C. METCALF ; B. N. GRAY.** *Biometals,* 2003, vol. 16, 455
- **T.-J. YOON ; H. LEE ; H. SHAO ; S. A. HILDERBRAND ; R. WEISSLEDER.** *Adv. Mater.,* 2011, vol. 23, 4793
- **J.-H. LEE ; Y.-W. JUN ; S.-I. YEON ; J.-S. SHIN ; J. CHEON.** *Angew. Chem.,* 2006, vol. 118, 8340
- *Angew. Chem. Int. Ed.,* 2006, vol. 45, 8160
- **J. E. LEE et al.** *J. Am. Chem. Soc.,* 2010, vol. 132, 552